# EUROPEAN PATENT APPLICATION

(11) **EP 4 647 001 A1**
(43) Date of publication of application: **12.11.2025**
(21) Application number: 25153261.0
(22) Date of filing: 22.01.2025
(51) Int. Cl.: A61B 5/01, A61B 5/11, A61B 5/117, A61B 5/145, A61B 5/0531

(54) **SYSTEM, BIOSENSOR DEVICE AND METHOD FOR MONITORING PHYSIOLOGICAL SIGNALS OF A USER**

(30) Priority: 22.01.2024 SE 2450059
(71) Applicant: Lantau Petersen, Benjamin, 7120 Vejle Ø (DK); SPT Vilecon A/S, 2640 Hedehusene (DK)
(72) Inventor: LANTAU PETERSEN, Benjamin, 7120 VEJLE Ø (DK)
(74) Representative: Ström & Gulliksson AB

(57) **Abstract**

System (10) for monitoring physiological signals of a user (12), comprising a biosensor carrier (14) and a biosensor device (16). The biosensor device (16) is configured to obtain physiological signals of the user (12) and location data of a position where the user (12) is located; set a plurality of sensing threshold limits, each sensing threshold limit indicating a limit value for causing an external transmission of at least the obtained physiological signals to an external device (20); and in response to at least one of the physiological signals violating a sensing threshold limit from among the plurality of sensing threshold limits, transmit said at least one of the physiological signals to the external device (20). The transmission is effected over a cellular network using a low-power communication protocol and further comprises the location data and a unique identifier associated with the biosensor device (16).

## Description

### TECHNICAL FIELD

The present invention relates to devices for monitoring physiological signals of a user. More particularly, the present invention relates to a system, a biosensor device and an associated method.

### BACKGROUND

Continuous health monitoring through biosensor systems has emerged as a transformative approach in healthcare. These systems encompass various devices aimed at tracking vital signs and physiological data, including heart rate, blood pressure, glucose levels, temperature, and more. However, prevalent biosensor technologies encounter challenges, for example related to energy efficiency, particularly in continuous monitoring applications. Traditional biosensor systems often rely on expensive and heavy battery-powered mechanisms for data acquisition, processing, and wireless transmission.

It is in view of these challenges and others that the present inventor herein suggests improvements to the prior art of biosensor systems.

### SUMMARY

An object of the present invention is therefore to provide a solution overcoming the disadvantages of prior art. More specifically, the present invention provides a solution involving monitoring physiological signals of a user.

In a first aspect, a system for monitoring physiological signals of a user is provided. The system comprises a biosensor carrier adapted to be removably attached to a skin of the user and a biosensor device arranged in the biosensor carrier. The biosensor device is configured to obtain physiological signals of the user and location data of a position where the user is located, set a plurality of sensing threshold limits, each sensing threshold limit indicating a limit value for causing an external transmission of at least the obtained physiological signals to an external device in response to at least one of the physiological signals violating a sensing threshold limit from among the plurality of sensing threshold limits, and transmit said at least one of the physiological signals to the external device. The transmission is effected over a cellular network using a low-power communication protocol and further comprises the location data and a unique identifier associated with the biosensor device.

In some embodiments, the plurality of sensing threshold limits are set based on personal characteristics of the user.

In some embodiments, the plurality of sensing threshold limits comprises a physiological signal limit.

In some embodiments, the plurality of sensing threshold limits comprises a network connectivity signal limit.

In some embodiments, the plurality of sensing threshold limits comprises a movement signal limit.

In some embodiments, the plurality of sensing threshold limits are adjustable based on a user activity and/or a user state.

In some embodiments, the biosensor device is further configured to set an adjustable user activity and/or user state.

In some embodiments, the plurality of sensing threshold limits, the user activity and/or the user state are adjustable through manual user input and/or automatic detection by the biosensor device.

In some embodiments, the transmission is effected based on a combination of one or more of the sensing threshold limits, the location data, a user activity, and a user state.

In some embodiments, the unique identifier involves low-power connectivity identification.

In some embodiments, the biosensor device comprises one or more sensors.

In some embodiments, the external device is a device of a caregiver, healthcare professional, emergency user, healthcare monitoring services, healthcare facilities, support network, health insurance provider, community health service, public health department, and/or a relative.

In some embodiments, the biosensor system is provided with a built-in sharing feature, a sharing application service allowing invited family members and/or caregivers to monitor user's data and/or trends, a sharing application notifying if threshold limits are passed and/or a service allowing the user to send SOS Help message to followers.

In some embodiments, the biosensor carrier is removably attached to the skin of the user by plastering, sandwiching or using an adhesive.

In some embodiments, the physiological signals are one or more of cardiac rhythm metrics, blood oxygenation metric, skin conductance metrics, respiratory rate metrics, muscle activity metrics, blood glucose metrics, hydration metrics and skin temperature metrics.

In some embodiments, the biosensor device is configured to obtain the location data through a locational service provider.

In some embodiments, the biosensor device is configured to detect whether the biosensor device is making direct contact with the skin across portions of the bottom surface of the biosensor device.

In some embodiments, the biosensor device is further configured to employ a radio-frequency harvesting technology.

In some embodiments, the biosensor system is a single-use system.

In some embodiments, the low-power communication protocol is one of Narrowband Internet of Things (NB-IoT) or a protocol used over a Low Power Wide Area Network (LPWAN).

In a second aspect a biosensor device is provided. The biosensor device is arranged in a biosensor carrier adapted to be removably attached to a skin of the user, and the biosensor device is configured to: obtain physiological signals of the user and location data of a position where the user is located; set a plurality of sensing threshold limits, each sensing threshold limit indicating a limit value for causing an external transmission of at least the obtained physiological signals to an external device; and in response to at least one of the physiological signals exceeding a sensing threshold limit from among the plurality of sensing threshold limits, transmit said at least one of the physiological signals to the external device, wherein the transmission is effected over a cellular network using a low-power communication protocol and further comprises the location data and a unique identifier associated with the biosensor device.

In a third aspect a method of monitoring physiological signals of a user is provided. The method comprises obtaining physiological signals of the user and location data of a position where the user is located, setting a plurality of sensing threshold limits, each sensing threshold limit indicating a limit value for causing an external transmission of at least the obtained physiological signals to an external device, and transmitting, in response to at least one of the physiological signals violating a sensing threshold limit from among the plurality of sensing threshold limits, said at least one of the physiological signals to an external device. The transmission is effected over a cellular network using a low-power communication protocol and further comprises the location data and a unique identifier associated with the biosensor device.

### BRIEF DESCRIPTION OF THE DRAWINGS

Embodiments of the invention will be described in the following description of the present invention; reference being made to the appended drawings which illustrate non-limiting examples of how the inventive concept can be reduced into practice.
FIG. 1 is a schematic view of a biosensor system according to one embodiment.
FIG. 2 is a schematic diagram of a biosensor system for communicating with an external device according to one embodiment.
FIG. 3A is a schematic view of a user scenario according to one example.
FIG. 3B is a schematic view of a user scenario according to one example.
FIG. 4 is a schematic view of a biosensor system attachment according to one embodiment.
FIG. 5 is a schematic block view of a method of monitoring a user health state.

### DETAILED DESCRIPTION

Embodiments of the invention will now be described with reference to the accompanying drawings. The invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the particular embodiments illustrated in the accompanying drawings is not intended to be limiting of the invention. In the drawings, like numbers refer to like elements.

The term "coupled" is defined as connected, although not necessarily directly, and not necessarily mechanically. Two or more items that are "coupled" may be integral with each other. The terms "a" and "an" are defined as one or more unless this disclosure explicitly requires otherwise. The terms "substantially", "approximately", and "about" are defined as largely, but not necessarily wholly what is specified, as understood by a person of ordinary skill in the art. The terms "comprise" (and any form thereof, such as "comprises" and "comprising"), "have" (and any form thereof, such as "has" and "having", "include" (and any form thereof, such as "includes" and "including") and "contain" (and any form thereof, such as "contains" and "containing") are open-ended linking verbs. As a result, a method that "comprises", "has", "includes" or "contains" one or more steps possesses those one or more steps, but is not limited to possessing only those one or more steps.

The energy consumption inefficiencies in prior biosensor systems manifest in various ways. The prior art commonly utilizes wireless communication protocols, such as Bluetooth, Wi-Fi, or proprietary transmission methods, to relay data from the biosensor to external receivers or monitoring devices. However, these transmission mechanisms tend to consume considerable power, leading to rapid depletion of the device's battery life. Some existing biosensor systems employ continuous data streaming for real-time monitoring purposes. Transmitting data in real-time necessitates consistent energy usage, significantly impacting the device's battery life and overall operational efficiency. With reliance on conventional battery-based power sources, these biosensor systems struggle with limited operational lifespan and frequent recharging or replacements. This dependency hampers prolonged usage without interruptions.

The present disclosure relates to a biosensor system for monitoring the health of a user. The biosensor system is configured to obtain physiological signals of the user, set sensing threshold limits which, if violated, causes transmission of at least the physiological signals to an external device. The notification may lead to more versatile assistance arriving to the user depending on various different circumstances pertaining to which types of sensing threshold limits being violated, to what extent, where, how, or the like. Furthermore, the system communicates using a low-power communication protocol, meaning low energy usage and a slow drainage of the battery. This may enable long time use and limited user administration compared to traditional systems. Interruptions to the continuous monitoring may thus be reduced. Improving the battery lifetime of a health-monitoring device can be the difference between a potentially life threatening condition being detected or not. The specific combination of providing a biosensor device that sets a plurality of threshold limits, transmits notifications to external device(s) once one or more of these limits are transgressed, bases the transmission(s) upon a low-power communication protocol over the cellular network, and provides location data as well as a unique identifier to the transmission, may be seen as particularly advantageous way of assessing a user's health status over a prolonged period of time.

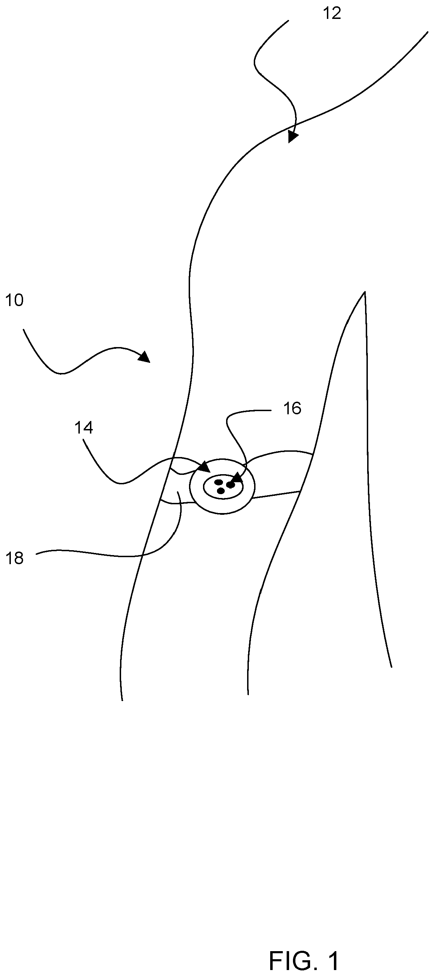

Starting in FIG. 1, a biosensor system 10 (or system 10, for reasons of brevity) is shown. The biosensor system 10 comprises a biosensor carrier 14 configured to hold a biosensor device 16. The biosensor carrier 14 is attached to a user 12. In this example the biosensor carrier 14 is attached to a user's 12 arm, although any other alternative placements may be envisaged in various other exemplary biosensor systems. In other non-limiting examples, the biosensor carrier 14 may be attached to a leg, abdomen, neck, torso, ear, foot, and so forth. The biosensor carrier 14 is in this example attached to the user 12 by a strap 18. Any other type of fasteners may be envisaged, such as a belt, band, clip, or the like. The carrier 14 may be configured to hold the biosensor device 16 to the user 12 using sandwiching, plastering or by means of an adhesive. The adhesive is preferably of a kind that is non-irritating for the user's skin, such as hypoallergenic adhesives, silicone-based adhesives, hydrocolloid adhesives, acryl-based adhesives, or the like. By way of the adhesive strength and the area that the biosensor carrier 14 fastens to, i.e., the skin, the biosensor carrier 14 is configured to sit tightly to the user 12 such that the biosensor carrier 14 is prohibited from accidentally fall off the user 12, yet is easily removable in a pain-free way. In this way, the biosensor carrier 14 is removably attached to the skin of the user, thereby enabling fast, easy and pain-free fastening or removal of the biosensor carrier 14 from the skin of the user 12.

The biosensor device 16 is further configured to obtain physiological signals and location data associated with the user 12. The biosensor device 16 can obtain the location data by way of a locational service provider, such as a GPS unit, cellular and/or Wi-Fi network, Bluetooth beacon, inertial measurement sensor, barometric pressure sensor, cellular assisted GPS unit, and/or IP geolocation units.

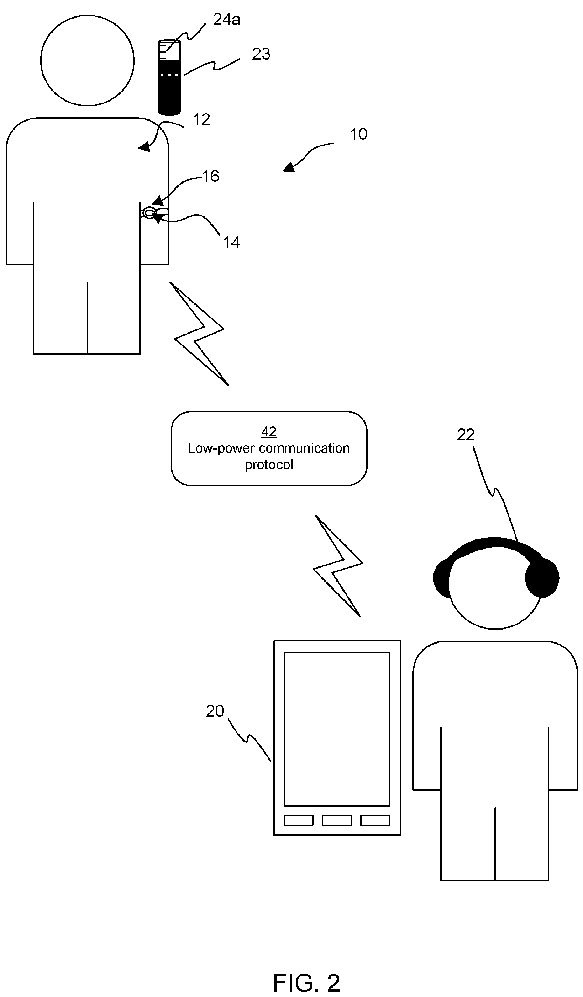

The biosensor device is configured to set a plurality of sensing threshold limits which, if violated, causes the transmission of at least the physiological signals to an external device from the biosensor device 16. The external device will be discussed in more detail later on with further reference to FIGs. 2-3, and could be any device capable of receiving physiological signals, location signals and user identification such as a stationary computer, laptop, smartphone, smart watch, or tablet.

In some embodiments, the sensing threshold limits comprise a physiological signal limit, a movement signal limit, and/or a network connectivity signal limit. The sensing threshold limits can pertain to a plurality of limits that might be of interest to persons or institutions, who will be later described, to whom the physiological signals are to be sent and/or shared with followers. For example, the physiological sensing threshold limits can be pertain to one or more of location based thresholds, temperature based thresholds, skin conductance based thresholds, EKG-based thresholds, EEG-based thresholds, EMG-based thresholds, blood pressure based thresholds, movement based thresholds, blood oxygen saturation thresholds, respiration rate based thresholds, blood glucose based thresholds, hydration based thresholds, and environmental factors based thresholds (e.g. environmental factors that may be unhealthy for the user 12, or in some embodiments that effect the other sensing threshold limits). The movement signal limits can pertain to one or more of acceleration based thresholds and speed based thresholds. The network connectivity thresholds may pertain to network conditions experienced by the biosensor device 16, including e.g. packet losses or download/upload speeds.

Violating a sensing threshold limit may involve a value of a physiological signal being either below a minimum sensing threshold limit, and/or above a maximum sensing threshold limit. In some examples there may be values for certain physiological signals that do not necessarily require a lower or upper limit, and in other examples the allowable values may be based on a range of values having a lower and upper limit, respectively. For example, a temperature-based sensing threshold limit may have both a maximum and minimum value, indicating that the user 12 has a fever (temperature above a maximum allowable value) or is hypothermic (temperature below a minimum allowable value).

Furthermore, the biosensor device 16 holds a unique identifier, indicating the identity of the user 12. This may enable a secure transmission of physiological signals to the external device 20, which is important during transmission of sensitive data such as physiological signals of the user 12. According to some embodiments, the unique identifier is one or more of a user device registration, a biometric identification, a device pairing via Bluetooth, a token-based identification, a digital certificate or key, and a user-provided identification, such as a scanned QR code or an image of a physical identification (such as a driver's license).

In some embodiments the system 10 is a single-use system. The biosensor system 10 is charged prior to fastening to the body. Such a single-use system could be particularly useful in a healthcare setting, elderly care setting, and/or elderly population system, as the users may then be ill-equipped to handle technology due to confusion, unfamiliarity or dementia. Furthermore, continuous monitoring may be especially necessary for users with an elevated risk of health-related issues.

In some embodiments the system 10 comprises a local storage unit. The local storage unit is configured to store historic data. The historic data may comprise information, like any of the physiological signals or location data, of the user 12 stored during the time which the user 12 carries the system 10. Alternatively, the system 10 comprises an external storage unit. The system 10 may be configured to wirelessly communicate with the external storage unit, such that the historical data can be stored and/or processed therein. The storage unit can include, but is not limited to, devices like a magnetic disk drive, a solid state drive, an optical drive, a flash memory card, a memory stick, etc. The storage unit may also include a cloud-based server implemented using any commonly known cloud-computing platform such as Amazon Web Services, Google Cloud Platform, Microsoft Azure, DigitalOcean, IBM Bluemix or Alibaba Cloud.

The biosensor device 16 may comprise a controller unit that may be implemented in any known controller technology, including but not limited to microcontroller, processor (e.g. PLC, CPU, DSP, FPGA, ASIC) or any other suitable digital and/or analogue circuitry capable of performing the intended functionality. It may further comprise a memory associated with the controller unit which may be implemented in any known memory technology, including but not limited to E(E)PROM, S(D)RAM or flash memory.

The biosensor device 16 may comprise a communication unit (e.g. transceiver) adapted to receive and transmit information (such as signals). The communication unit is configured to communicate using a low-power communication protocol. Low-power communication protocols known in the art may be based on Low Power Wide Area Network technologies, such as LoRaWAN, Sigfox or Thread. The low-power communication protocol may alternatively be Narrowband Internet of Things (NB-IoT). These low-power communication protocol may further improve the lifetime of the biosensor device 16, and thus the biosensor system 10. The use of a low-power communication protocol, such as NB-IoT, is particularly advantageous as it may decrease the need to recharge or switch devices thanks to an increased battery life. This can be of utmost importance to, for example, users suffering from a confused state or dementia, as they might forget to put on the biosensor system 10 after removal for charging or switching.

The biosensor device 16 may comprise a rechargeable battery. In one embodiment, the rechargeable battery is recharged using radio frequency (RF) harvesting technology, for instance using piezo- or triboelectric energy harvesting or by utilizing a rectenna (rectifying antenna), which may prolong the time during which the user 12 can be monitored by the biosensor device 16. Prior art systems often lack efficient energy harvesting mechanisms to supplement or replenish power reserve, which typically leads to reliance on removal of the biosensor system in order to allow it to recharge the batteries, or frequent switching between products. As a result, the shortcomings in energy efficiency and power management of existing biosensor systems restrict their viability for sustained continuous monitoring. This embodiment may be advantageous in this regard.

The present inventor has realized surprisingly advantageous effects provided by a biosensor device 16 having a battery being rechargeable by way of employing RF energy harvesting, in combination with the low-power communication protocol as discussed herein. Not only can the biosensor device 16 be operative for a plurality of days without needing replacement, the biosensor device 16 and thus the entire system 10 consequently also has a low weight, is cheap, and easy to attach to the skin of the user 12. This may make the system 10 particularly advantageous as single-use systems, for example provided by healthcare professionals for monitoring physiological signals of the user 12 through a limited, although prolonged, period of time, such as up to one week. Moreover, the present inventor has also realized that this combined embodiment works particularly well in contexts where the transmission of the physiological signals is effected over the cellular network. The integration of the low-power communication protocol into the cellular network infrastructure may leverage the existing cellular ecosystem and thus provides even lower deployment costs. This also offers a high reliability in terms of signal strength, particularly given upcoming rollouts of emerging cellular communication technologies including e.g. 5G or 6G.

The biosensor device 16 may be further configured to detect if it is properly attached to the skin of the user 12. This may be done using a skin-detection sensor. The skin-detection sensor may be one or more of a light emitting and receiving sensor, a capacitive sensor, a pressure sensor, an optical sensor, a temperature sensor, a strain gauge, an ultrasound sensor, a biopotential sensor, an adhesive sensor, or the like. The light emitting and receiving sensor, and/or optionally any of the other sensors discussed above, may be capable of detecting if the biosensor device 16 is making direct contact with the skin across portions of the bottom surface of the biosensor device 16. The portions of the bottom surface may include at least substantially the entirety of the bottom surface.

Turning to FIG. 2, a biosensor system 10 according to one embodiment is shown. In this example, an otherwise healthy user 12, wearing the biosensor system 10 has temperature in excess of a set sensing threshold limit 23.

In the prevailing example, the physiological signals are temperature signals. As the temperature signals 24a detected by the biosensor device 16 are in excess of the sensing threshold limit 23 the biosensor device sends the temperature signal as well as a location data indicating the position of the user 12 to an external device 20. The user 12 is identified by the external device 20 by means of the earlier aforementioned unique identifier. The device 20 could be a device of a caregiver and/or follower, who can administer help to the user 12 with the elevated temperature.

In some embodiments, the sensing threshold limits of the user 12 has a uniqueness attribute, which is based on the individual characteristics of the user 12. This may be obtained from the historical data over time, as discussed in the example above. Alternatively, the biosensor device 16 may be configured to obtain the uniqueness attribute from a medical record (optionally inserted by a healthcare professional or the user). The uniqueness attribute may alternatively be obtained from other inputs, such as behavioural biometrics from how the user 12 operates the device, genetic information of the user 12, environmental interactions (e.g. location data of where the user is typically located and how it affects physiological signals), or the like. Other uniqueness attributes not explicitly accounted for herein may alternatively be envisaged. **In** the exemplary FIG. 2 the user 12 could have a detected normal body temperature of 37.2 C, and the uniqueness attribute pertaining to body temperature may be 37.2 °C. **In** that case, the threshold limit pertaining to temperature for causing transmission of the physiological signals to the external device 20 may be adapted in accordance with the uniqueness attribute. By way of these embodiments, inadvertent transmission of signals can be prevented, which may improve the lifetime of the biosensor device 16, as well as preventing unnecessary safety measures to be put in place where they are not necessarily required.

Causing the external notification may entail notifying and/or contacting one or more followers, such as a caregiver, healthcare professional, emergency user, healthcare monitoring services, healthcare facilities, caregivers or support networks, health insurance providers, community health services or public health departments, and/or a relative, via the built-in sharing application. Different types of transmissions to different users may be sent based various different conditions. Such conditions may include one or more of whether a threshold limit has been violated, which threshold limit that has been violated, to what extent a threshold limit has been violated, how many threshold limits have been violated, and where a user is located (e.g. based on the location data) when a certain threshold limit has been violated. In one embodiment, the external notification transmission is effected by the level of which a threshold has been exceeded. For example, if a body temperature of the user 12 exceeds a temperature threshold limit by a relatively lower amount (such as 0.1 °C or 0.2 °C), an external device 20 of a follower, such as a relative or a local caregiver, can be notified via the transmission of the external signal. If, on the other hand, the body temperature of the user 12 exceeds the temperature threshold limit by relatively higher amount (such as 1.0 °C or 1.5 °C), it may be necessary to transmit the physiological signals and location data to a hospital instead. The biosensor device 16 may further indicate directly to the user 12 that a sensing threshold limit has been violated, for example via haptic feedback (e.g. through a piezoelectric actuator or other such actuators) or auditory feedback (e.g. via a speaker or headphones 22).

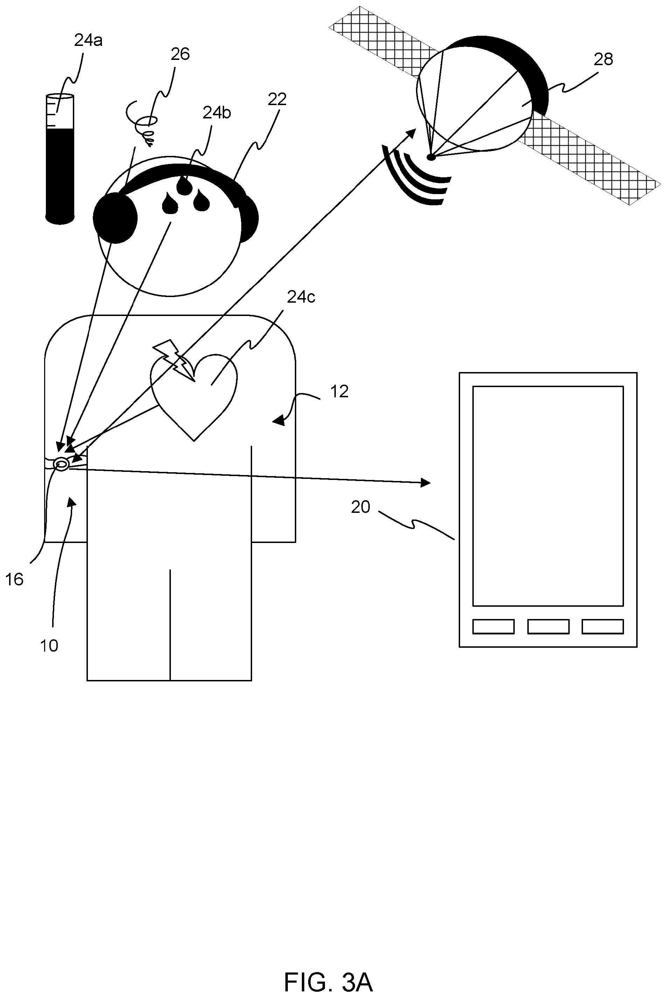

FIG. 3A shows an example of an implementation of the biosensor system 10. In this example, the biosensor device 16 has set a plurality of physiological limits comprising a temperature threshold limit 24a, a skin conductance limit 24b and a heart rate limit 24c. Furthermore, the biosensor device 16 has obtained location data from a satellite unit 28, and a user state 26 indicating dementia. In more general examples, the user state may be a mental state associated with the user, such as a mental health diagnosis, a general mental state (e.g. confusion or anger management issues), prior health issues, or some other chronic or temporary state. In the prevailing example, one or more of the physiological limits has been violated, and as such at least one notification involving at least the physiological signals that have violated the corresponding physiological limits have been transmitted to an external device 20.

In some embodiments, the transmission is effected based on a combination of one or more of the sensing threshold limits, the location data, a user activity, and a user state. In these embodiments, even if one or more sensing threshold limits have been violated, the biosensor device 16 does not necessarily cause an external notification since the user activity and/or the user state may not allow it. Alternatively, even if a sensing threshold limit has not been violated, the external notification may still be transmitted. For example, if the biosensor device 16 is indicating that the user 12 is exercising but has not yet reached an elevated heart rate, and the user 12 has a previous history of heart issues, the biosensor device 16 may notify a follower, such as a caregiver or relative, in an attempt to make him stop the exercise routine. Alternatively, the external device 20 may give the user 12 instructions to stop the exercise routine, such as through a pair of headphones. In another example, a combination of sensing threshold limits may be analysed to determine if the external device 20 is to be notified, and if a follower, i.e. an external user of the external device 20, shall be notified. For example, if the user 12 only has a fever a relative may be contacted. If there's a fever as well as a low respiratory rate, an emergency caregiver may be contacted.

In one embodiment, the sensing threshold limits, a user state, and a user activity are adjustable. The user 12 may make such an adjustment through interaction with the biosensor device 16 or through interaction with an external device, such as a computer program product (e.g. software program) associated therewith. Alternatively, the adjustment may take place by another person through an external device. For example, a healthcare expert may adjust the sensing threshold limits to account for new knowledge around the health status of the user 12. For example, the user 12 might be in a location far from home as indicated by location data obtained from the satellite unit 28. Due to the user 12 also having a user state 26 indicating dementia, the sensing threshold limit may be adjusted to cause a transmission to an external device of the physiological signals and location data if the user 12 ventures outside of a home environment. The biosensor device 16 may thus communicate the location data to an appropriate external device 20, for example a device operated by a follower, such as a relative. The follower, such as a relative, may make physical contact with the user 12 and help the user 12 home. In a further embodiment, another external device not shown in FIG. 3A, or the biosensor device 16 itself, may communicate to the user 12 that his or her location has been transmitted to the appropriate external device 20.

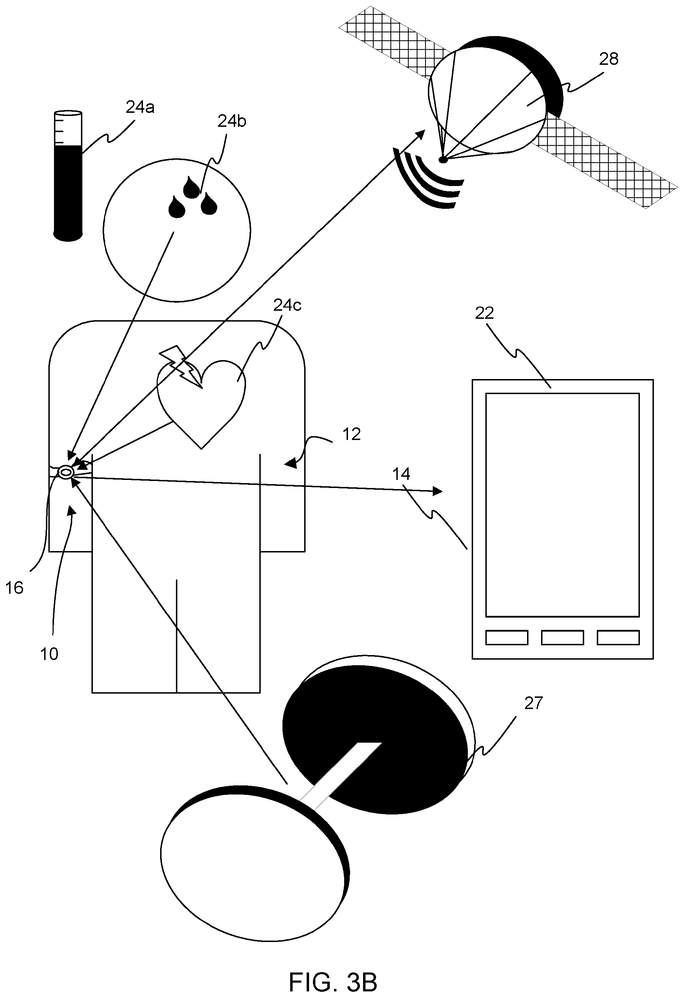

Turning to FIG. 3B, another example is shown where the user 12 is no longer suffering from dementia and has instead indicated a user activity, in this case exercising with a dumbbell 27. In this example, the threshold limits have been adjusted according to the obtained user activity.

In some embodiments, the inputs of user activities and/or user states are carried out by automatic detection by and/or manual input to the biosensor device 16. Manual input may include input from any of the aforementioned operators of the external device 20, or from the user 12 through a user interface of an external device or of the biosensor device. Automatic detection may include determining, by the biosensor device 16, a user state or user activity based on acquired physiological signals, movement signals, network connectivity signals, or location data. For example, if the user 12 is detected to be rapidly moving up and down, and is located in a training environment, the user activity may be adjusted to indicate a training activity. As such, a sensing threshold limit associated with vertical movement can be adjusted.

By using a combination of sensing threshold limits, user activity, or user state, or by adjusting the same, the system 10 is flexible and properly adjusts, in some embodiments automatically, to the user 12 of the biosensor device 16. This leads to improvements related to the monitoring of a person's health status, as the individual needs of the user are holistically taken into account.

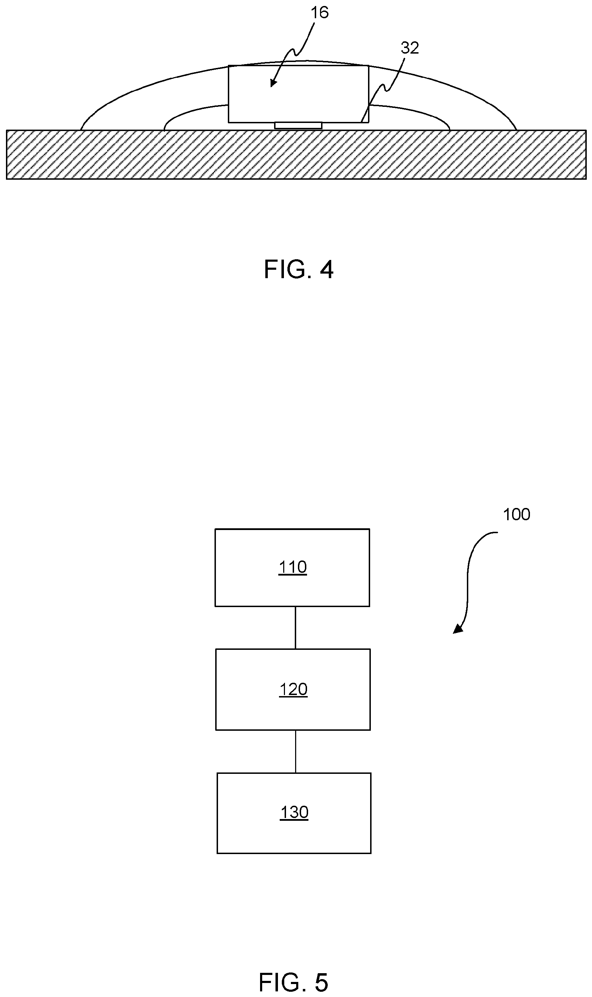

FIG. 4 shows the biosensor carrier 16 attached to a user by means of plastering and adhesive. A bottom side 32 of the biosensor carrier 16 is coated with an adhesive, which improves the connection between the biosensor system and the user skin.

FIG. 5 shows a schematic block diagram of a method 100 of causing one or more external transmissions of at least the physiological signals when a user could be indicating a risk to their health. The method 100 comprises obtaining 110 physiological signals of the user and location data of a position where the user is located. The method 100 further comprises setting 120 a plurality of sensing threshold limits, each sensing threshold limit indicating a limit value for causing an external transmission of at least the obtained physiological signals to an external device. The method 100 further comprises transmitting 130, in response to at least one of the physiological signals violating a sensing threshold limit from among the plurality of sensing threshold limits, said at least one of the physiological signals to an external device. The transmission is effected over a cellular network using a low-power communication protocol and further comprises the location data and a unique identifier associated with the biosensor device.

Although the present invention has been described above with reference to specific embodiments, it is not intended to be limited to the specific form set forth herein. Rather, the invention is limited only by the accompanying claims.

## Claims

1. System (10) for monitoring physiological signals of a user (12), the system (10), comprising:
a biosensor carrier (14) adapted to be removably attached to a skin of the user (12); and
a biosensor device (16) arranged in the biosensor carrier (14), the biosensor device (16) being configured to:
- obtain physiological signals of the user (12) and location data of a position where the user (12) is located;
- set a plurality of sensing threshold limits, each sensing threshold limit indicating a limit value for causing an external transmission of at least the obtained physiological signals to an external device (20); and
- in response to at least one of the physiological signals violating a sensing threshold limit from among the plurality of sensing threshold limits, transmit said at least one of the physiological signals to the external device (20),
wherein the transmission is effected over a cellular network using a low-power communication protocol and further comprises the location data and a unique identifier associated with the biosensor device (16).

2. The system (10) according to claim 1, wherein the plurality of sensing threshold limits are set based on personal characteristics of the user (12).

3. The system (10) according to any of the preceding claims, wherein the plurality of sensing threshold limits comprises a physiological signal limit.

4. The system (10) according to any of the preceding claims, wherein the plurality of sensing threshold limits comprises a network connectivity signal limit.

5. The system (10) according to any of the preceding claims, wherein the plurality of sensing threshold limits comprises a movement signal limit.

6. The system (10) according to any of the preceding claims, wherein the plurality of sensing threshold limits are adjustable based on a user activity and/or a user state.

7. The system (10) according to any of the preceding claims, wherein the biosensor device (16) is further configured to set an adjustable user activity and/or user state.

8. The system (10) according to claim 7, wherein the plurality of sensing threshold limits, the user activity and/or the user state are adjustable through manual user input and/or automatic detection by the biosensor device (16).

9. The system (10) according to any of the preceding claims, wherein the transmission is effected based on a combination of one or more of the sensing threshold limits, the location data, a user activity, and a user state.

10. The system (10) according to any of the preceding claims, wherein the unique identifier involves low-power connectivity identification.

11. The system (10) according to any of the preceding claims, wherein the biosensor device (16) comprises one or more sensors.

12. The system (10) according to any of the preceding claims, wherein the external device (20) is a device of a caregiver, healthcare professional, emergency user, healthcare monitoring services, healthcare facilities, support network, health insurance provider, community health service, public health department, and/or a relative.

13. The system (10) according to any of the preceding claims, wherein the external device (20) is provided with a built-in sharing service application.

14. The system (10) according to any of the preceding claims, wherein the biosensor carrier (14) is removably attached to the skin of the user (12) by plastering, sandwiching or using an adhesive.

15. The system (10) according to any of the preceding claims, wherein the physiological signals are one or more of cardiac rhythm metrics, blood oxygenation metric, skin conductance metrics, respiratory rate metrics, muscle activity metrics, blood glucose metrics, hydration metrics and skin temperature metrics.

16. The system (10) according to any of the preceding claims, wherein the biosensor device (16) is configured to obtain the location data through a locational service provider.

17. The system (10) according to any of the preceding claims, wherein the biosensor device (16) is configured to detect whether the biosensor device (16) is making direct contact with the skin across portions of the bottom surface of the biosensor device (16).

18. The system (10) according to any of the preceding claims, wherein the biosensor device (16) is further configured to employ a radio-frequency harvesting technology.

19. The system (10) according to any of the preceding claims, the biosensor system (10) being a single-use system.

20. The system (10) according to any of the preceding claims, wherein the low-power communication protocol is one of Narrowband Internet of Things (NB-IoT) or a protocol used over a Low Power Wide Area Network (LPWAN).

21. A biosensor device (16) arranged in a biosensor carrier (14) adapted to be removably attached to a skin of the user (12), the biosensor device (16) being configured to:
obtain physiological signals of the user (12) and location data of a position where the user (12) is located;
set a plurality of sensing threshold limits, each sensing threshold limit indicating a limit value for causing an external transmission of at least the obtained physiological signals to an external device (20); and
in response to at least one of the physiological signals exceeding a sensing threshold limit from among the plurality of sensing threshold limits, transmit said at least one of the physiological signals to the external device (20),
wherein the transmission is effected over a cellular network using a low-power communication protocol and further comprises the location data and a unique identifier associated with the biosensor device (16).

22. A method (100) of monitoring physiological signals of a user, the method (100), comprising:
- obtaining (110) physiological signals of the user and location data of a position where the user is located;
- setting (120) a plurality of sensing threshold limits, each sensing threshold limit indicating a limit value for causing an external transmission of at least the obtained physiological signals to an external device; and
- transmitting (130), in response to at least one of the physiological signals violating a sensing threshold limit from among the plurality of sensing threshold limits, said at least one of the physiological signals to an external device,
wherein the transmission is effected over a cellular network using a low-power communication protocol and further comprises the location data and a unique identifier associated with the biosensor device.
